# EUROPÄISCHE PATENTSCHRIFT

(11) **EP 0 602 274 B1**
(45) Veröffentlichungstag und Bekanntmachung des Hinweises auf die Patenterteilung: **09.04.1997**
(21) Anmeldenummer: 92121599.2
(22) Anmeldetag: 18.12.1992
(51) Int. Cl.: A61F 2/36, A61F 2/30, A61F 2/46, A61F 2/44

(54) **Knochenimplantat mit Hohlraum**
Bone implant having a cavity
Implant osseux comportant une cavité

(43) Veröffentlichungstag der Anmeldung: 22.06.1994
(60) Teilanmeldung: 96111669.6
(73) Patentinhaber: Härle, Anton, Prof. Dr., D-48161 Münster (DE)
(72) Erfinder: Härle, Anton, Prof. Dr., D-48161 Münster (DE)

(56) Entgegenhaltungen:
- EP-A- 0 268 115
- EP-A- 0 315 283
- EP-A- 0 428 127
- EP-A- 0 434 604
- EP-A- 0 487 865
- EP-A- 0 516 569
- WO-A-93/08769
- DE-A- 2 751 832
- DE-A- 3 704 089
- DE-A- 4 122 950
- DE-U- 9 213 218
- US-A- 3 426 364
- US-A- 4 994 085
- US-A- 5 116 380

## Beschreibung

Die Erfindung bezieht sich auf ein Knochenimplantat mit einer mit einer Saugleitung versehenen Vorrichtung zur Erzeugung eines Unterdrucks zur Ansaugung von Knochenzement.

Endoprothesen als in den Körper verbrachte Implantate zur Defektauffüllung nach Gewebeentfernungen oder künstliche Gelenke erfordern für eine gute Funktionsfähigkeit eine ausreichende Stabilisierung im umgebenden, körpereigenen Knochengewebe. Die Implantation von Fremdkörpern geht mit einer erhöhten Infektionsrate einher, wobei durch die Infektion eine Lockerung des Implantats ausgelöst werden kann und wobei die Infektion an sich eine zum Teil lebensbedrohende Komplikation darstellt.

Bei der Verankerung der Endoprothese besteht die Möglichkeit, eine sogenannte "Preß-Fit-Verankerung" vorzunehmen, bei der eine Kongruenz zwischen Implantatlager, also dem körpereigenen Knochen, und der Prothesenform besteht. Die Anforderung an das Implantatlager sowie an die Operationstechnik sind hierbei hoch. Außerdem stellt der sehr unterschiedliche Elastizitätsmodul bei dem Knochengewebe einerseits und dem metallischen Implantat andererseits ein nur schwer kompensierbares Problem bei der Endoprothetik dar.

Die oben genannten Nachteile werden bei der Zementierung des Implantats im Knochen vermieden, da die Knochenzementschicht zwischen Knochen und Metallimplantat ähnlich einem Puffer wirkt und so das Implantatlager schützt.

Bei der Zementierung ist nachteilig, daß Inhomogenitäten des Knochenzements die theoretisch möglichen vorteilhaften Effekte teilweise aufheben können. Das portionsweise Einbringen von Knochenzement führt immer zu Luft- und Blutbeimischungen, so daß der Knochenzement lamelliert und mechanisch geschwächt wird.

Weiterhin kommt es durch das nachträgliche Einschieben der Endoprothese, insbesondere an Krümmungsstellen des Knochens, zu Abschabungen des Knochenzements vom Knochenlager, so daß an dieser Stelle die Prothese überhaupt nicht vom Knochenzement umschlossen ist. Belastungsspitzen sowohl an der Prothese wie auch an den Knochenzementgrenzen sind dann zwangsläufig, wobei diese Belastungsspitzen wiederum Brüche des Knochenzements oder auch der Prothese selbst nach sich ziehen können.

Weiterhin entsteht beim Einpressen des Knochenzements ein hoher Überdruck in der Markhöhle, so daß es zu Fettausschwemmungen aus dem Knochenmark kommt, die zu Embolien führen können. Aus diesem Grund wird teilweise der Knochenzement durch Unterdruck in den Knochenhohlraum eingesaugt.

Zu diesem Zweck wird unterhalb des von der Prothese später auszufüllenden Bereichs eine Saugöffnung in den Knochen gebohrt, wobei an dieser Saugöffnung ein Unterdruck angelegt werden kann, der den Knochenzement von der Implantationsstelle des Knochens her in den Knochenhohlraum ansaugt.

Nachteilig dabei ist, daß das Einbringen der Endoprothese selbst immer noch zu einer Druckerhöhung im Markraum führt. Weiterhin können die Knochenzementhinterschneidungen und damit die späteren Hohlräume im Knochenzement nicht vermieden werden, da die gekrümmten Knochenhohlräume beim Einbringen der Prothese ein Abschaben des vorher eingebrachten Knochenzements begünstigen.

Ein weiterer Nachteil dieser Methode besteht darin, daß zur Unterdruckerzeugung unterhalb der später eingebrachten Prothese ein Bohrloch im Knochen in dem Abschnitt erforderlich ist, der zur Knochenmitte hin gerichtet ist. Dieses Bohrloch stellt eine mechanische Beeinträchtigung und damit eine Frakturgefährdung für den Knochen dar. Außerdem muß dieser Bohrkanal weitab vom eigentlichen Operationsgebiet angelegt werden, so daß entweder eine Verlängerung der Operationswunde oder eine zusätzliche Operationswunde erforderlich werden.

Der Erfindung liegt die Aufgabe zugrunde, einen dauerhaft festen Sitz des Knochenimplantats sicherzustellen und gleichzeitig einen möglichst schonenden Operationsverlauf zu ermöglichen.

Diese der Erfindung zugrundeliegende Aufgabe wird durch die Ausgestaltung eines Knochenimplantats mit einer mit einer Saugleitung versehenen Vorrichtung zur Erzeugung eines Unterdrucks zur Ansaugung von Knochenzement gemäß Anspruch 1 gelöst.

Weitere vorteilhafte Ausgestaltungen können den Unteransprüchen entnommen werden.

Die Erfindung schlägt mit anderen Worten vor, das Knochenimplantat mit einem durchgehenden Hohlraum auszustatten, der Sauganschlüsse aufweist, so daß der Knochenzement durch die Prothese angesaugt werden kann, d.h. dann angesaugt werden kann, wenn die Prothese sich schon in ihrer eingesetzten optimalen Stellung befindet. Das Einsaugen des Knochenzements um die lagerichtig angeordnete Prothese stellt eine homogene Knochenzementschicht mit den entsprechenden mechanischen Vorteilen sicher.

Auf diese Weise ist eine Verfahrensweise bei der Implantation von Endoprothesen möglich, wie sie beispielsweise aus dem Stahlbetonbau bekannt ist:

Zunächst werden tragende Elemente in ihrer mechanisch optimalen Lage angeordnet. Schließlich werden diese Elemente mit einer Sekundärumgießung umgeben, so daß sich ein idealer Verbund ergeben kann. Nur durch eine besondere Formgestaltung der Prothese kann dieses Prinzip umsetzbar gemacht werden.

Gemäß einem weiteren Merkmal der Erfindung kann das Knochenimplantat zusätzliche Öffnungen aufweisen, durch die in die Hohlkörper eingebrachte Pharmaka Wirkstoffe z. B. als Zytostatika oder Antibiotika an das die Prothese umgebende Gewebe abgeben können. Auf diese Weise wird nicht nur die möglicherweise lebensbedrohliche Komplikation durch Infektionen verringert oder verhindert, sondern auch die mechanisch stabile und dauerhafte Halterung der Prothese im umgebenden Knochenzement und dem darum angeordneten Knochengewebe begünstigt.

Dabei kann eine Ummantelung vorgesehen sein, die die zusätzlichen Öffnungen der Prothese bei einem in der Prothese anliegenden Unterdruck abdichtet und auf diese Weise die Saugwirkung durch den Hohlraum in der Prothese sicherstellt. Die Ummantelung kann dabei scharfkantig ausgebildete Bereiche der Prothese abdecken und einen mechanischen Schutz für das umliegende Gewebe bieten. Zusätzlich kann die Ummantelung thermisch isolierende oder sogar kühlende Eigenschaften aufweisen, um das umliegende Gewebe vor einer thermischen Schädigung zu schützen, wenn bei der Polymerisation des Knochenzements Temperaturerhöhungen von bis zu 90° auftreten.

Gemäß einem weiteren Merkmal der Erfindung kann die dauerhafte, mechanische Stabilität der Prothesenverankerung dadurch gesichert werden, daß zusätzliche Befestigungsmittel an bzw. in der Prothese vorgesehen sind. Zu diesem Zweck können sich Laschen von der eigentlichen Prothese an die benachbarten Knochenbereiche erstrecken, oder es kann an bzw. in der Prothese eine Aufnahme für Schrauben oder ähnliche Befestigungsmittel vorgesehen sein.

Gemäß einem letzten Gesichtspunkt der Erfindung kann die dauerhafte und sichere Fixierung der Prothese dadurch begünstigt werden, daß die Steifigkeit der Prothese entsprechend beeinflußt wird. Hier ist insbesondere eine Anpassung des Elastizitätsmoduls der Prothese an den Elastizitätsmodul des umgebenden Knochengewebes günstig. Zu diesem Zweck kann der Prothesenhohlraum mit einer dreidimensional gitterartigen Innenstruktur versehen sein oder der Prothesenhohlraum kann mit Stoffen der geeigneten mechanischen Eigenschaften verfüllt werden und so zu einer Verfestigung der Prothese beitragen.

Diese Stoffe können vorteilhaft Pharmaka enthalten und beispielsweise als Antibiotika oder Zytostatika ihre medizinische Wirkung entfalten. Um Belastungsspitzen zu vermeiden und auf diese Weise für eine dauerhaft sichere Fixierung der Prothese zu sorgen, kann die Prothese in bestimmten Fällen auch aus zwei oder mehr Komponenten bestehen, die gelenkig miteinander verbunden sind, so daß die Prothese nur gewisse Kräfte aufnehmen muß, während die Gelenke für eine entsprechende Nachgiebigkeit in anderen Belastungsrichtungen sorgen.

Weitere vorteilhafte Ausgestaltungen der Erfindung sind aus den Ansprüchen zu entnehmen.

Ausführungsbeispiele eines erfindungsgemäßen Implantates sind in den Zeichnungen näher beschrieben. Dabei zeigt
- Fig. 1: ein Femurimplantat einer Hüftprothese in perspektivischer und teilweise geschnittener Ansicht,
- Fig. 2: einen Querschnitt durch den Gegenstand von Fig. 1,
- Fig. 3: einen Querschnitt ähnlich Fig. 2 durch ein zweites Ausführungsbeispiel und
- Fig. 4: ein Wirbelsäulenimplantat in perspektivischer Ansicht.

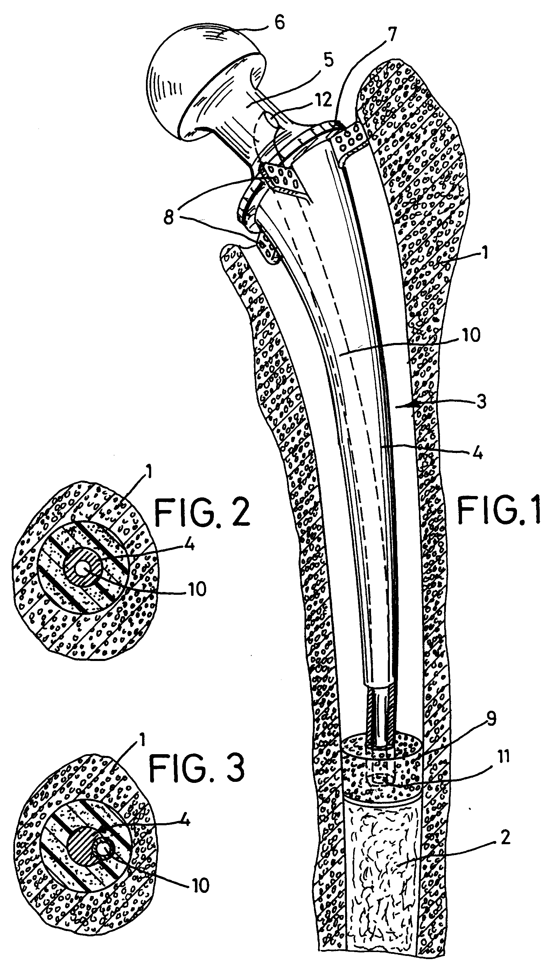

In Fig. 1 ist mit 1 ein Oberschenkelknochen (Femur) bezeichnet, der in seinem Inneren Knochenmark 2 aufweist. Im oberen Bereich des Oberschenkelknochens 1 ist das Knochenmark 2 entfernt worden und mit einem Fräs- oder Raspelwerkzeug ein Hohlraum mit genau definierter Form geschaffen worden.

In diesem Hohlraum befindet sich ein Implantat 3, das aus einem Schaft 4, einem Hals 5 und einem Kopf 6 besteht. Im Übergangsbereich zwischen dem Schaft 4 und dem Hals 5 ist ein Kragen 7 angeformt. Zentriervorsprünge 8 fixieren das Implantat 3 lagerichtig innerhalb des Oberschenkelknochens 1. Die Zentriervorsprünge 8 sind als einzelne Zentrierfinger unterhalb des Kragens 7 ausgestaltet. In Abwandlung von dem dargestellten Ausführungsbeispiel ist es auch möglich, anstelle der Zentrierfinger den Kragen 7 zu einem Zentrierkragen weiterzubilden, der über Einfüllöffnungen für Knochenzement verfügt. Die Zentriervorsprünge können entweder an dem Implantat fest angeformt sein oder entfernbar ausgebildet sein, so daß sie beispielsweise zur lagerichtigen Fixierung der Prothese zunächst an der Prothese befestigt werden. Dann wird die Prothese mit den Fixierungen lagerichtig eingesetzt und einzementiert und anschließend werden diese oberen Fixiervorsprünge wieder entfernt.

Im unteren Bereich wird die Prothese 3 durch einen Stopfen 9 zentriert, der porös oder mit Kanälen versehen ist.

Die Prothese 3 weist über nahezu ihre gesamte Länge einen durchgehenden Hohlraum 10 in Form einer Durchgangsbohrung auf, wobei die Prothese 3 an ihrem untersten Ende innerhalb des Stopfens 9 eine untere Öffnung 11 und im Bereich des Halses 5 eine seitliche, obere Öffnung 12 aufweist, die beide durch den Hohlraum 10 miteinander verbunden sind. Diese Öffnungen stellen Sauganschlüsse dar, mit deren Hilfe innerhalb des Knochens ein Unterdruck angelegt werden kann.

Durch die Zentrierung wird gewährleistet, daß die Prothese 3 überall einen nahezu gleichmäßigen Abstand zum umgebenden Oberschenkelknochen 1 aufweist. Dieser, in etwa rohrförmige Hohlraum soll durch Knochenzement ausgefüllt werden, um einen guten und sicheren sowie dauerhaften Sitz der Prothese 3 innerhalb des Knochens 1 zu gewährleisten.

Der Knochenzement wird zu diesem Zweck um den Kragen 7 herum zugeführt, während an der oberen Öffnung 12 ein Unterdruck erzeugt, also gesaugt wird. Der Unterdruck breitet sich von dem oberen Sauganschluß (Öffnung 12) durch den Hohlraum 10 und den unteren Sauganschluß (Öffnung 11) sowie durch die Poren oder Kanäle innerhalb des Stopfens 9 aus, so daß anschließend auch im rohrförmigen Hohlraum um die Prothese 3 herum ein Unterdruck entsteht. Auf diese Weise wird von oben nachgeführter Knochenzement um die Prothese 3 herum in den Knochen 1 eingesaugt.

Wenn der Knochenzement unten am Stopfen 9 angelangt ist, können zwei verschiedene Vorgehensweisen verwendet werden:

Bei einem feinporigen Stopfen 9 kann über den erhöhten Saugwiderstand festgestellt werden, daß der Knochenzement unten am Stopfen 9 anliegt. Aufgrund seiner Viskosität kann der Knochenzement nicht in den Stopfen 9 eindringen. Die Zufuhr weiteren Knochenzementes wird unterbunden. Der Knochenzement kann nun abbinden und gewährleistet nach dem Abbinden den erwünschten sicheren Sitz der Prothese im Knochen.

Der Prothesenhohlraum kann unausgefüllt verbleiben oder mit Material gefüllt werden, welches erwünschte mechanische und/oder pharmakologische Eigenschaften aufweist.

Alternativ dazu kann ein großporiger Stopfen verwendet werden oder ein Stopfen, der in seinem oberen Bereich Ausnehmungen aufweist, die eine Verbindung von dem rohrförmigen Hohlraum um die Prothese 3 herum zu dem unteren Sauganschluß (Öffnung 11) schaffen. Bei geeigneter Größe dieser Ausnehmungen oder Poren kann angesaugter Knochenzement durch den Stopfen 9 in die untere Öffnung 11 und damit in den Hohlraum 10 eingesaugt werden, im Hohlraum 10 ansteigen und schließlich an der oberen Öffnung 12 austreten.

Der Austritt des Knochenzementes an der oberen Öffnung 12 stellt dabei einen sicheren Indikator dafür dar, daß der Hohlraum um die Prothese 3 herum vollständig mit Knochenzement ausgefüllt ist. Dabei können dem Knochenzement pharmakologische Wirkstoffe zugegeben sein, die Infektionen innerhalb des Hohlraumes 10 und an den Öffnungen 11 und 12 verhindern.

Welche der beiden Befüllungsmethoden angewandt wird, hängt davon ab, ob der Hohlraum 10 mit Knochenzement ausgefüllt werden soll oder nicht. Um einen dauerhaft guten Sitz der Prothese innerhalb des Knochens zu gewährleisten, ist es vorteilhaft, daß die Prothese einen Elastizitätsmodul aufweist, der dem des umgebenden Knochens möglichst ähnlich ist. Durch die Auswahl des Materials für die Prothese, für den Zement, und durch die Wahl, ob in der Prothese ein Hohlraum 10 verbleibt oder dieser Hohlraum mit Zement verfüllt wird, sind verschiedene Anpassungen und verschiedene Ausgestaltungen der Elastizitätsmodule möglich.

Weiterhin wird durch die Zentrierung der Prothese eine gleichmäßig dicke und überall ausreichend dicke Knochenzementschicht um die Prothese herum gewährleistet, so daß die Zementschicht nicht an bestimmten, zu dünnen Stellen brechen kann. Ein Bruch der Zementschicht schädigt nicht allein die Zementschicht selbst, sondern führt im längeren Verlauf auch zu Schädigungen der benachbarten Knochenpartien, so daß durch die gleichmäßige Zementschicht in Folge der zentrierten Lage der Prothese ein zweifacher Vorteil erzielt wird:

Zunächst bietet die gleichmäßige Ausfüllung des Hohlraumes um die Prothese eine optimale mechanische Kraftübertragung von der Prothese auf den umgebenden Knochen und zweitens wird der resorbierende Angriff durch Granulationsgewebe auf den Knochen unterbunden, der im Bereich von Stör- oder Bruchstellen des Knochenzementes üblicherweise auftritt.

Der Stopfen 9 kann zusammen mit der Prothese 3 in den Knochenhohlraum eingebracht werden. Beim späteren Einbringen der Prothese kann die entsprechende Formgebung von Zentrierelement und Prothesenspitze dafür Sorge tragen, daß die Prothese lagerichtig innerhalb des Knochens fixiert wird.

Soll abweichend von dem dargestellten Ausführungsbeispiel ein Ansaugen von Knochenzement nicht durch die Prothese selbst erfolgen, sondern durch eine seitliche Bohrung, die möglichst tief an bzw. unterhalb der Prothese durch den Knochen verläuft, so kann es vorteilhaft sein, an dem zunächst eingebrachten Zentrierelement einen Indikator anzuordnen, der außerhalb des Knochens die Lage des Zentrierelementes anzeigt. So kann z. B. das Zentrierelement an einem Stab befestigt sein und an diesem Stab in den Knochen eingeführt werden, wobei parallel zu diesem Stab ein zweiter Stab angeordnet ist, der an seiner Spitze außerhalb des Knochens die Lage des Zentrierelementes innerhalb des Knochens anzeigt. Ein derartiger Indikator ist im wesentlichen U-förmig ausgebildet und ermöglicht es, die Saugbohrung durch den Knochen in das Zentrierelement zu richten.

Wenn der Unterdruck am Bereich der oberen Öffnung 12 aufgebaut wird, wirkt dieser Unterdruck über die Kanäle oder Poren des Stopfens 9. Um ein Ansaugen des Knochenmarks 2 zu verhindern, kann der Stopfen 9 an seiner Unterseite abdichtend ausgebildet werden, so daß der Unterdruck nicht über den Stopfen 9 hinaus auf das Knochenmark 2 einwirkt. Auf diese Weise wird verhindert, daß Knochenmark 2 in den Stopfen 9 eingesaugt wird.

Je nach Viskosität des verwendeten Knochenzementes und des Knochenmarks 2 kann diese abdichtende untere Schicht des Stopfens 9 auch entfallen, wenn der Unterdruck lediglich ein Ansaugen des Knochenzements bewirkt, jedoch ein Eindringen des Knochenmarks 2 in den Stopfen 9 nicht zuläßt. Dies hängt vom Zusammenspiel der Viskosität des verwendeten Knochenzementes, der Viskosität des Knochenmarks 2, der Poren- oder Kanalgröße im Stopfen 9 und der Stärke des angelegten Unterdruckes ab.

Fig. 2 zeigt einen Querschnitt durch die Ansicht von Fig. 1 im Bereich des Schaftes 4 der Prothese, wobei abweichend zu der Fig. 1 der Bereich zwischen dem Knochen 1 und dem Schaft 4 mit Knochenzement ausgefüllt dargestellt.

Abweichend von dem in Fig. 1 und 2 dargestellten Ausführungsbeispiel kann der Hohlraum auch seitlich an der Prothese angeordnet sein. Er kann einerseits in den Schaft (4) eingeformt sein oder - wie in Fig. 3 angedeutet - in eine Einbuchtung des Schaftes (4) eingepaßt sein und als separates Bauteil beispielsweise in Rohrform ausgebildet sein. Dabei kann das Rohr als entfernbarer Hohlkörper nach der Operation an dem Schaft (4) verbleiben und hohl bleiben oder gefüllt werden oder er kann nach dem Einbringen der Prothese und des Knochenzementes entfernt werden.

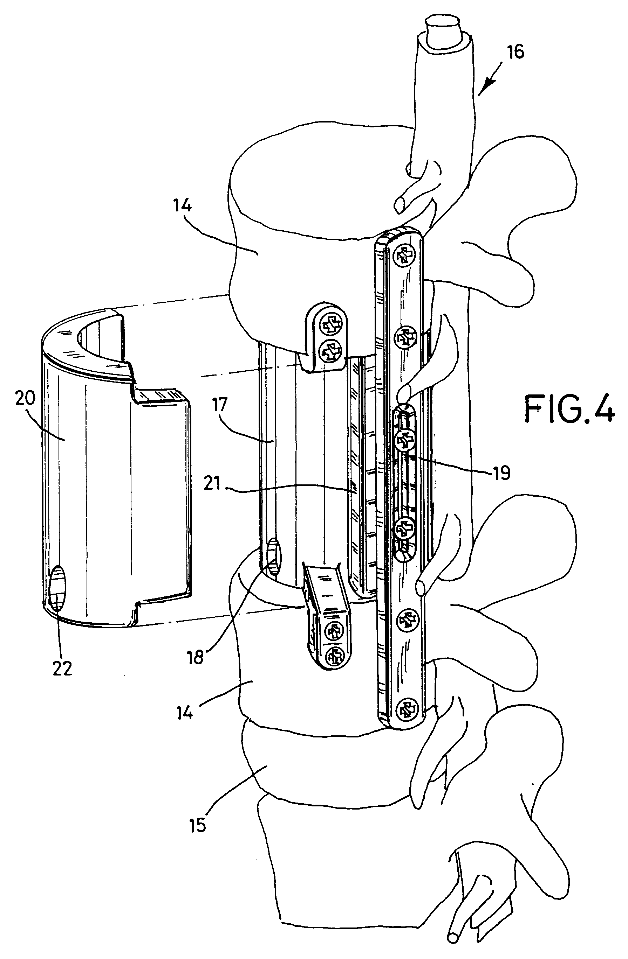

Fig. 4 zeigt eine Wirbelsäulenprothese, wobei schematisch eine Wirbelsäule mit mehreren Wirbelknochen 14, Bandscheiben 15 und einem Rückenmark 16 dargestellt ist. Eine rohrförmige Prothese 17 ist zwischen zwei Wirbelknochen 14 eingesetzt und weist ebenfalls zwei Öffnungen zum Einsaugen des Knochenzementes auf, wobei von den beiden lediglich eine Öffnung 18 dargestellt ist.

Neben der Prothese 17 sind Fixierungsplatten 19 vorgesehen, die durch Schrauben an ihren beiden Enden die beiden durch die Prothese 17 verbundenen Wirbelknochen 14 gegen Verdrehungen festlegen. Weiterhin weisen die Fixierungsplatten 19 in ihrem mittleren Bereich Langlöcher auf, durch die sich Schrauben in die Prothese 17 zu deren achsengerechter Fixierung erstrecken. Die Prothese 17 kann zu diesem Zweck vorbereitete Aufnahmen für die Schrauben aufweisen.

Alternativ hierzu oder zusätzlich sind an der Prothese 17 Laschen vorgesehen, die sich in den Bereich des benachbarten gesunden Knochengewebes erstrecken und eine Fixierung der Prothese 17 direkt an den benachbarten Wirbelknochen 14 ermöglichen.

Beim Abbinden des Knochenzements treten unerwünscht hohe Temperaturen auf, die das empfindliche Rückenmark 16 möglicherweise schädigen könnten. Es ist daher bei derartigen Wirbelsäulenoperationen notwendig, das Rückenmark 16 gegen die Hitzeentwicklung des abbindenden Knochenzementes zu schützen. Zu diesem Zweck ist ein zweiteiliger Mantel schematisch dargestellt, der aus zwei Mantelhälften 20 und 21 besteht. Die Mantelhälften weisen Öffnungen 22 auf, durch welche die Einlaß- bzw. die Auslaßöffnung 18 der Prothese erreichbar sind, so daß die-Prothese 17 bei angelegtem Mantel mit Knochenzement gefüllt werden kann. Der Mantel kann dabei isolierende Eigenschaften aufweisen oder ein Kühlmittel enthalten oder Kanäle für den Durchfluß eines Kühlmittels enthalten.

Der Mantel ist aus den Mantelhälften 20 und 21 aufgebaut, damit er nach der Abkühlung des Knochenzementes entfernt werden kann. Die abgeschlossene Wandung der Hohlprothese 17 und die Entfernbarkeit des Mantels stellen sicher, daß nach Einbringen der Prothese 17 eine definierte Ausdehnung des Knochenzementes sichergestellt ist, so daß weder der Knochenzement noch - durch die Entfernbarkeit - der Mantel das Rückenmark schädigen kann. Ein entsprechend weicher Mantel kann ggf. auch an der Prothese verbleiben, ohne daß nahe Gewebebereiche geschädigt werden.

Auch in der Prothese 17 kann der eingebrachte Knochenzement pharmakologische Wirkstoffe enthalten, die als Antibiotikum oder Zytostatikum wirken. Dabei kann die Prothese 17 Öffnungen aufweisen, die den Durchtritt dieser Wirkstoffe durch die Prothesenwandung zum Gewebe ermöglichen. Die Prothese 17 kann dazu z. B. gitter- oder netzartig ausgebildet sein.

Die Ummantelung trägt in diesem Fall dafür Sorge, daß zunächst nur die Sauganschlüsse frei sind. Nach Einsaugen des Knochenzements und ggf. nach dessen Abkühlung wird die Ummantelung entfernt, so daß die Pharmaka auf das umliegende Gewebe einwirken können.

## Patentansprüche

1. Knochenimplantat mit einer mit einer Saugleitung versehenen Vorrichtung zur Erzeugung eines Unterdrucks zur Ansaugung von Knochenzement, mit einem Schaft und einem den Schaft (4) durchsetzenden Kanal (10), der in eine Öffnung ( 11, 18) in einem ersten Endbereich des Schaftes (4) mündet, und mit einer am anderen Ende des Kanals befindlichen zweiten Öffnung (12) an der Außenseite des Implantats (3, 17), wobei sich der Kanal (10) in Implantatslängsrichtung durch den gesamten Schaft (4) des Knochenimplantats (3, 17) erstreckt, wobei die zweite Öffnung des Kanals als ein Saugleitungsanschluß (12) zum Anschließen der Saugleitung ausgebildet ist und entweder in einem dem ersten Endbereich gegenüberliegenden zweiten Endbereich dieses Schaftes oder noch weiter vom ersten Endbereich entfernt außerhalb des Schaftes (4) angeordnet ist, wobei das Knochenimplantat (3, 17) im Schaftbereich (4) zwischen der Öffnung (11, 18) im ersten Endbereich und dem Saugleitungsanschluß (12) eine durchgehende Mantelfläche aufweist, die keine von der Außenseite bis zum Kanallumen reichenden Poren oder Öffnungen aufweist.

2. Knochenimplantat nach Anspruch 1, dadurch gekennzeichnet, daß der Kanal entweder als Bohrung durch den Schaftbereich ausgebildet ist oder eine eigene Wandung aufweist.

3. Knochenimplantat nach Anspruch 1, dadurch gekennzeichnet, daß der Kanal (10) zentral in dem Implantat (3, 17) angeordnet ist.

4. Knochenimplantat nach Anspruch 1, dadurch gekennzeichnet, daß der Kanal (10) seitlich im Implantat ausgebildet ist.

5. Knochenimplantat nach 1, gekennzeichnet durch einen von dem Implantat entfernbaren Kanal.

6. Knochenimplantat nach einem der Ansprüche 1 bis 3, gekennzeichnet durch ein Zentrierelement (9) am Endbereich des Implantats.

7. Knochenimplantat nach einem der Ansprüche 1 bis 4, gekennzeichnet durch ein frittenartiges Zentrierelement (9) im Bereich der Implantatspitze, wobei der implantatferne Bereich des Zentrierelementes (9) undurchlässig und der implantatnahe Bereich des Zentrierlementes (9) durchlässig ausgebildet ist.

8. Knochenimplantat nach einem der Ansprüche 1 bis 6, gekennzeichnet durch ein oder mehrere Zentrierelemente (8) im Bereich der Implantationsstelle des Knochens (1).

9. Knochenimplantat nach einem der Ansprüche 5 bis 8, gekennzeichnet durch als Zentrierfinger ausgebildete Zentrierelemente (8).

10. Knochenimplantat nach einem der Ansprüche 5 bis 8, gekennzeichnet durch ein als Zentrierkragen ausgebildetes Zentrierelement.

11. Knochenimplantat nach einem der Ansprüche 5 bis 8, gekennzeichnet durch ein als Zentrierstopfen (9) ausgebildetes Zentrierelement.

12. Knochenimplantat nach einem der Ansprüche 5 bis 11, gekennzeichnet durch ein von dem Implantat (3) lösbar ausgebildetes Zentrierelement (8).

13. Knochenimplantat nach einem der Ansprüche 5 bis 12, gekennzeichnet durch ein seitliches Bohrloch in dem Zentrierelement sowie durch einen Indikator, der die Lage des im Knochen befindlichen Zentrierelementes und des Bohrloches außen am Knochen anzeigt.

14. Knochenimplantat nach Anspruch 13, gekennzeichnet durch einen im wesentlichen U-förmigen Indikator, dessen einer Schenkel in den Knochen zum Zentrierelement verläuft und dessen anderer Schenkel außerhalb des Knochens verläuft.

15. Knochenimplantat nach einem der vorhergehenden Ansprüche, dadurch gekennzeichnet, daß die zwischen der Öffnung (11, 18) im erstgenannten Endbereich und dem Saugleitungsanschluß (12) befindliche Mantelfläche als eine ablösbare Ummantelung (20, 21) ausgebildet ist, welche Öffnungen im Kern des von ihr ummantelten Knochenimplantats (17) bei dem im Knochenimplantat (17) herrschenden Unterdruck abdichtend abdeckt und lediglich Saugöffnungen (18) freiläßt, welche mit Sauganschlüssen in Verbindung treten.

16. Knochenimplantat nach Anspruch 15, gekennzeichnet durch Öffnungen im Kern des Knochenimplantats, die den Durchtritt pharmazeutischer Wirkstoffe aus dem Hohlraum im Knochenimplantat (10) zum umliegenden Gewebe (1, 14, 15, 16) ermöglichen.

17. Knochenimplantat nach einem der vorhergehenden Ansprüche, gekennzeichnet durch eine wärmeisolierende Ummantelung (20, 21).

18. Knochenimplantat nach einem der vorhergehenden Ansprüche, gekennzeichnet durch eine Ummantelung (20, 21) mit einem Kühlsystem und Anschlüssen für die Zufuhr von Kühlmittel.

19. Knochenimplantat nach einem der vorhergehenden Ansprüche, gekennzeichnet durch eine entfernbar ausgebildete Ummantelung (20, 21).

20. Knochenimplantat nach einem der vorhergehenden Ansprüche, gekennzeichnet durch eine mehrteilig ausgebildete Ummantelung (20, 21).

21. Knochenimplantat nach einem der vorhergehenden Ansprüche, gekennzeichnet durch Verankerungsmittel für Befestigungsmittel.

22. Knochenimplantat nach Anspruch 21, gekennzeichnet durch an die Prothese angeformte Laschen zur Aufnahme von Schrauben, Nägeln, Stiften und ähnlichen Befestigungsmitteln, wobei sich die Laschen von der Prothese (17) aus erstrecken.

23. Knochenimplantat nach Anspruch 21, gekennzeichnet durch im Bereich der Prothese vorgesehene Aufnahmen für Nägel, Stifte, Schrauben und ähnliche Befestigungsmittel.

24. Knochenimplantat nach einem der vorhergehenden Ansprüche, gekennzeichnet durch einen Hohlraum, der eine netzartige Innenstruktur zur Erhöhung der mechanischen Belastbarkeit der Prothese aufweist.

25. Knochenimplantat nach einem der vorhergehenden Ansprüche, dadurch gekennzeichnet, daß der Hohlraum ein Pharmaka enthaltendes und freisetzendes Material enthält oder zur Aufnahme solcher Pharmaka ausgebildet ist.

26. Knochenimplantat nach einem der vorhergehenden Ansprüche, dadurch gekennzeichnet, daß der Hohlraum ein die Prothese stabilisierendes Verfestigungsmaterial enthält.

## Claims

1. A bone implant comprising a device provided with a suction channel for creating negative pressure for the aspiration of bone cement, comprising a shank and a channel (10) passing through the shank (4), which channel (10) opens in an opening (11, 18) in a first end area of the shank (4), and comprising a second opening (12) located at the other end of the channel on the outside of the implant (3, 17), the channel (10) extending in the longitudinal direction of the implant through the entire shank (4) of the bone implant (3, 17), the second opening in the channel being constructed as a suction channel connection (12) for connecting the suction channel and being arranged either in a second end area of this shank opposite the first end area or even further from the first end area outside the shank (4), the bone implant (3, 17) comprising a continuous surface area in the shank region (4) between the opening (11, 18) in the first end area and the suction channel connection (12), which surface area does not comprise any pores or openings extending from the outside to the channel opening.

2. A bone implant according to claim 1, characterised in that the channel is constructed either as a bore through the shank area or comprises its own inherent wall.

3. A bone implant according to claim 1, characterised in that the channel (10) is arranged centrally in the implant (3, 17).

4. A bone implant according to claim 1, characterised in that the channel (10) is constructed laterally in the implant.

5. A bone implant according to claim 1, characterised by a channel removable from the implant.

6. A bone implant according to any one of claims 1 to 3, characterised by a centring element (9) at the end area of the implant.

7. A bone implant according to any one of claims 1 to 4, characterised by a frit-type centring element (9) in the region of the implant tip, the area of the centring element (9) remote from the implant being impermeable and the area of the centring element (9) near the implant being permeable.

8. A bone implant according to any one of claims 1 to 6, characterised by one or more centring elements (8) in the area of the implantation point of the bone (1).

9. A bone implant according to any one of claims 5 to 8, characterised by centring elements (8) constructed as centring fingers.

10. A bone implant according to any one of claims 5 to 8, characterised by a centring element constructed as a centring collar.

11. A bone implant according to any one of claims 5 to 8, characterised by a centring element constructed as a centring stopper (9).

12. A bone implant according to any one of claims 5 to 11, characterised by a centring element (8) constructed so as to be detachable from the implant (3).

13. A bone implant according to any one of claims 5 to 12, characterised by a lateral hole in the centring element and by an indicator which indicates the position of the centring element located in the bone and the hole external to the bone.

14. A bone implant according to claim 13, characterised by a substantially U-shaped indicator, one leg of which extends into the bone towards the centring element and the other leg of which extends outside the bone.

15. A bone implant according to any one of the preceding claims, characterised in that the surface area located between the opening (11, 18) in the first end area and the suction channel connection (12) is constructed as a detachable casing (20, 21), which sealingly covers openings in the core of the bone implant (17) encased thereby when negative pressure prevails in the bone implant (17) and only releases suction openings (18) which are connected with suction connections.

16. A bone implant according to claim 15, characterised by openings in the core of the bone implant which enable the passage of pharmaceutically active substances out of the cavity in the bone implant (10) into the surrounding tissue (1, 14, 15, 16).

17. A bone implant according to any one of the preceding claims, characterised by a heat-insulating casing (20, 21).

18. A bone implant according to any one of the preceding claims, characterised by a casing (20, 21) with a cooling system and connections for the supply of coolant.

19. A bone implant according to any one of the preceding claims, characterised by a casing (20, 21) of removable construction.

20. A bone implant according to any one of the preceding claims, characterised by a casing (20, 21) of multi-part construction.

21. A bone implant according to any one of the preceding claims, characterised by anchoring means as fastening means.

22. A bone implant according to claim 21, characterised by tabs formed on the prosthesis for the accommodation of screws, nails, pins and like fastening means, the tabs extending out from the prosthesis (17).

23. A bone implant according to claim 21, characterised by receptacles provided in the area of the prosthesis for nails, pins, screws and like fastening means.

24. A bone implant according to any one of the preceding claims, characterised by a cavity comprising a mesh-type inner structure to increase the mechanical loadability of the prosthesis.

25. A bone implant according to any one of the preceding claims, characterised in that the cavity contains a material containing and releasing a pharmaceutical or is constructed to accommodate such pharmaceuticals.

26. A bone implant according to any one of the preceding claims, characterised in that the cavity contains a strengthening material stabilising the prosthesis.

## Revendications

1. Implant osseux ayant un dispositif comportant une conduite d'aspiration destinée à provoquer une dépression visant à aspirer du ciment orthopédique, une tige et un conduit (10) traversant la tige (4), débouchant dans une ouverture (11, 18) dans une première zone d'extrémité de la tige (4), et une deuxième ouverture (12) située à l'autre extrémité du conduit sur la face extérieure de l'implant (3, 17), où le conduit (10) se prolonge dans le sens longitudinal de l'implant à travers toute la tige (4) de l'implant osseux (3, 17), où la deuxième ouverture du conduit est formée comme raccord de conduite d'aspiration (12) destiné à raccorder la conduite d'aspiration et est disposée dans une deuxième zone d'extrémité de la tige en face de la première zone d'extrémité ou elle est éloignée encore plus de la première zone d'extrémité à l'extérieur de la tige (4), où l'implant osseux (3, 17) dans la zone de la tige (4) présente une surface d'enveloppe continue entre l'ouverture (11, 18) dans la première zone d'extrémité et le raccord de conduite d'aspiration (12), l'enveloppe ne présentant aucun pore ou aucune ouverture pénétrant de la face extérieure jusqu'au centre du conduit.

2. Implant osseux selon la revendication 1, caractérisé en ce que le conduit est formé comme alésage dans la zone de la tige ou présente une paroi propre.

3. Implant osseux selon la revendication 1, caractérisé en ce que le conduit (10) est centré dans l'implant (3, 17).

4. Implant osseux selon la revendication 1, caractérisé en ce que le conduit (10) est disposé latéralement dans l'implant.

5. Implant osseux selon la revendication 1, caractérisé par un conduit amovible par rapport à l'implant.

6. Implant osseux selon l'une des revendications 1 à 3, caractérisé par un élément de centrage (9) dans la zone d'extrémité de l'implant.

7. Implant osseux selon l'une des revendications 1 à 4, caractérisé par un élément de centrage (9) de type fritté dans la zone de la pointe de l'implant, où la zone de l'élément de centrage (9) éloignée de l'implant est imperméable et la zone de l'élément de centrage (9) proche de l'implant est perméable.

8. Implant osseux selon l'une des revendications 1 à 6, caractérisé par un ou plusieurs éléments de centrage (8) dans la zone du site d'implantation de l'os (1).

9. Implant osseux selon l'une des revendications 5 à 8, caractérisé par un élément de centrage (8) façonné sous forme d'ergot de centrage.

10. Implant osseux selon l'une des revendications 5 à 8, caractérisé par un élément de centrage façonné sous forme de collet de centrage.

11. Implant osseux selon l'une des revendications 5 à 8, caractérisé par un élément de centrage façonné sous forme de bouchon de centrage (9).

12. Implant osseux selon l'une des revendications 5 à 11, caractérisé par un élément de centrage (8) amovible par rapport à l'implant (3).

13. Implant osseux selon l'une des revendications 5 à 12, caractérisé par un trou latéral percé dans l'élément de centrage ainsi que par un indicateur permettant d'indiquer sur la face extérieure de l'os la position de l'élément de centrage dans l'os et du trou d'alésage.

14. Implant osseux selon la revendication 13, caractérisé par un indicateur de forme essentiellement en U dont une branche se prolonge dans l'os jusqu'à l'élément de centrage et dont l'autre branche se prolonge hors de l'os.

15. Implant osseux selon l'une des revendications précédentes, caractérisé en ce que la surface d'enveloppe située entre l'ouverture (11, 18) dans ladite première zone d'extrémité et le raccord de la conduite d'aspiration (12) est façonnée comme une enveloppe détachable (20, 21) qui recouvre de façon étanche les ouvertures dans le coeur de l'implant osseux (17) entouré par cette enveloppe lors de l'application de dépression dans l'implant osseux (17) et qui dégage uniquement les ouvertures d'aspiration (18) qui sont reliées à des raccords d'aspiration.

16. Implant osseux selon la revendication 15, caractérisé par des ouvertures au coeur de l'implant osseux, qui rendent possible la pénétration de principes actifs pharmaceutiques depuis la cavité dans l'implant osseux (10) jusqu'aux tissus environnants (1, 14, 15, 16).

17. Implant osseux selon l'une des revendications précédentes, caractérisé par une enveloppe d'isolation thermique (20, 21).

18. Implant osseux selon l'une des revendications précédentes, caractérisé par une enveloppe (20, 21) comportant un système refroidissant et des raccords d'introduction d'agent de refroidissement.

19. Implant osseux selon l'une des revendications précédentes, caractérisé par une enveloppe prévue amovible (20, 21).

20. Implant osseux selon l'une des revendications précédentes, caractérisé par une enveloppe conçue en plusieurs parties (20, 21).

21. Implant osseux selon l'une des revendications précédentes, caractérisé par des moyens d'ancrage en tant que moyens de fixation.

22. Implant osseux selon la revendication 21, caractérisé par des languettes placées sur la prothèse afin de recevoir des vis, clous, broches et moyens analogues de fixation, les languettes se prolongeant à partir de la prothèse (17).

23. Implant osseux selon la revendication 21, caractérisé par des sites prévus dans la zone de la prothèse pour la réception de clous, broches, vis et moyens de fixation analogues.

24. Implant osseux selon l'une des revendications précédentes, caractérisé par une cavité qui présente une structure interne réticulaire destinée à renforcer la capacité de charge mécanique de la prothèse.

25. Implant osseux selon l'une des revendications précédentes, caractérisé en ce que la cavité contient un matériau contenant et libérant un produit pharmaceutique ou qu'elle est conçue pour recevoir un tel produit pharmaceutique.

26. Implant osseux selon l'une des revendications précédentes, caractérisé en ce que la cavité contient un matériau de consolidation destiné à stabiliser la prothèse.
